# EUROPEAN PATENT SPECIFICATION

(11) **EP 3 265 144 B1**
(45) Date of publication and mention of the grant of the patent: **14.08.2019**
(21) Application number: 16759318.5
(22) Date of filing: 29.02.2016
(51) Int. Cl.: A61B 17/34, A61M 1/00, A61B 90/30, A61B 17/02, A61B 90/00

(54) **SURGICAL CANNULA WITH CHANNELS FOR IRRIGATION AND SUCTION**
CHIRURGISCHE KANÜLE MIT KANÄLEN ZUM SPÜLEN UND ABSAUGEN
CANULE CHIRURGICALE COMPRENANT DES CANAUX POUR L'IRRIGATION ET L'ASPIRATION

(30) Priority: 02.03.2015 US 201514635781
(43) Date of publication of application: 10.01.2018
(73) Proprietor: Warsaw Orthopedic, Inc., Warsaw, IN 46582 (US)
(72) Inventor: TONTZ, William, Minneapolis, Minnesota 55432 (US)
(74) Representative: Viering, Jentschura & Partner mbB Patent- und Rechtsanwälte
(86) International application number: PCT/US2016/020018
(87) International publication number: WO 2016/140905

(56) References cited:
- US-A- 5 407 427
- US-A1- 2007 027 364
- US-A1- 2010 262 080
- US-A1- 2010 262 080
- US-B1- 6 238 373
- US-B2- 8 226 553

## Description

### BACKGROUND OF THE INVENTION

The present invention relates to a surgical cannula used during the performance of a surgical procedure on a patient. More particularly, the present invention relates to a surgical cannula which is configured to spread open inside the patient to provide the surgeon with more unimpeded access to the surgical site; is configured to be held in place within the patient's body with limited, if any, assistance of external structures; is configured to limit a depth of insertion into the patient's body; and is configured with channels to light the operating site, and to irrigate and remove from the surgical site irrigation liquid and other unwanted materials.

### Description of the Prior Art

Surgical cannulas are known to provide access to a surgical site, e.g., a human spine. Such cannulas can be inserted into the patient's body during a surgical procedure either from an anterior approach or a posterior approach to provide access to the surgical site. Conventional surgical cannulas, however, have several disadvantages.

For example, the diameters of conventional cannulas typically are too small to provide surgeons with unimpeded access to surgical sites.

As another example, in order to hold a conventional surgical cannula in a desired position with respect to a patient's body during a surgical procedure, external clamping arms are required. Such external clamping arms hold the conventional cannula in position at one end thereof, and clamp to an operating table at the other end thereof. Use of such external clamping arms requires the patient to be placed under full anesthesia, since movement by the patient during the surgical procedure can cause the clamping arms to move, or even partially dislodge, the conventional cannula.

Additional shortcomings with the conventional surgical cannulas include: (1) it is sometimes difficult for surgeons to clearly see the respective surgical sites through the cannulas; (2) it is difficult to provide irrigation liquid to the surgical site; (3) when burrs are used to remove interfering bone near the respective surgical site, smoke, bone dust, and sometimes blood and other tissue further obscure visibility of the surgical site.

Example of prior art are given in e.g. US 2010/262080 A1, which discloses methods and devices being provided to improve access to a surgical site during surgical procedures, wherein in one exemplary embodiment a surgical access device is provided having a cannula, a cannula base, and a retractor, wherein the cannula base is configured to couple both to the retractor and the cannula to provide access to a surgical site, wherein the cannula can be removably and replaceably coupled to the cannula base, wherein, optionally, the cannula base can be removably and replaceably coupled to the retractor, wherein, as desired, the cannula can be decoupled from the cannula base, which can allow for an object to be removed from the surgical site and/or for the cannula, or another cannula to be attached to the cannula base that remains in place to maintain access to the surgical site, wherein exemplary methods for accessing a surgical site are also provided.

### SUMMARY OF THE INVENTION

In accordance with one aspect the present invention, a surgical cannula for accessing a surgical site in a body of a patient includes a cannula body having a proximal end, a distal end, and a peripheral surface. The cannula body has a first cannula body portion and a second cannula body portion, the first and second cannula body portions being pivotally attached to one another adjacent the proximal end. The cannula body also has a bore extending from the proximal end to the distal end, and a length between the proximal end and the distal end such that the cannula body extends from a superficial fascia of the patient above the surgical site to the surgical site.

The surgical cannula further includes a flange portion attached to the cannula body at the proximal end. The flange portion has an upper surface and a lower surface, and at least one aperture defined therebetween.

The surgical cannula yet further includes a thread around at least a portion of the peripheral surface of the cannula body. The thread, upon insertion of the cannula into the patient's body, is capable of holding the cannula in a desired position in the patient's body, and requires limited, if any, use of additional external structures.

The surgical cannula still further includes at least one channel in communication with the at least one aperture in the flange. The at least one channel extends from the flange portion through the cannula body to at least adjacent the distal end of the cannula body such that the at least one channel extends to at least adjacent the surgical site. A surgeon can use the at least one channel to: (1) provide additional light to the surgical site; (2) provide irrigation liquid to the surgical site; and/or (3) provide suction from the surgical site in order to remove irrigation liquid, bone dust, or other undesirable materials obscuring the visibility of the surgical site.

Further, a method of providing access to a surgical site in a body of a patient is provided as an example. The exemplary method includes utilizing a surgical cannula, the surgical cannula including a cannula body having a proximal end, a distal end, a central longitudinal axis extending through the proximal and distal ends, and a peripheral surface, the cannula body having a first cannula body portion and a second cannula body portion, the first and second cannula body portions being pivotally attached to one another adjacent the proximal end, the cannula body having a bore extending therethrough, and a length between the proximal end and the distal end such that the cannula body is adapted to extend from at least a superficial fascia of the patient above the surgical site to the surgical site, a flange portion attached to the proximal end of the cannula body, the flange portion having an upper surface, a lower surface, and at least a first aperture defined therebetween, a thread around at least a portion of the peripheral surface of the cannula body, the thread being adapted to hold the cannula in place in the patient's body, and at least a first elongated channel in communication with the first aperture in the flange, the first channel extending from the flange portion through the cannula body to at least adjacent the distal end of the cannula body such that the first channel is adapted to extend to at least adjacent the surgical site; inserting the cannula body, in an undeployed position, with the first and second cannula body portions proximate one another, into the body of the patient, at least until the lower surface of the flange portion contacts the superficial fascia of the patient's body, and the thread engages the patient's body; and providing one of light, irrigation, and suction to the surgical site via the first aperture and the first channel in communication therewith, wherein the cannula body, when in the undeployed position in the patient's body, provides, via the bore, a first amount of access to the surgical site.

Still further, another method of providing access to a surgical site of a body of a patient is provided as another example. The exemplary method including accessing the body of the patient above the surgical site; inserting a surgical cannula into the body of the patient, the surgical cannula having a body portion and a flange portion, the body portion having a first portion, a second portion, a proximal end, an opposite distal end, the first and second portions being moveable from an undeployed position to a deployed position, and the flange portion being positioned adjacent the proximal end; positioning the distal end of the body portion adjacent the surgical site with the first and second body portions in the undeployed position; accessing the surgical site through a bore formed through the surgical cannula; moving the first and second body portions from the undeployed position to the deployed position, the undeployed position providing a first amount of access to the surgical site, the deployed position providing a second amount of access to the surgical site greater than the first amount of access to the surgical site; providing a first one of light, irrigation, and suction to the surgical site via a first elongated channel through the body portion, the first elongated channel extending through the body portion from adjacent the flange portion to adjacent the distal end of the body portion; and providing a second one of light, irrigation, and suction to the surgical site via a second elongated channel through the body portion, the second elongated channel extending through the body portion from adjacent the flange portion to adjacent the distal end of the body portion. It is understood that both the foregoing general description and the followingdetailed description are exemplary and explanatory only, and are not restrictive of the invention as claimed.

### BRIEF DESCRIPTION OF THE DRAWINGS

The accompanying drawings, which are incorporated in and constitute a part of the specification, illustrate preferred embodiments of the invention and, together with the description, serve to explain the objects, advantages, and principles of the invention. In the drawings:
FIG. 1 is a top perspective view of a body of a patient, having a surgical cannula, in accordance with the present invention, inserted therein;
FIG. 2 is a top perspective view of a surgical cannula in accordance with the present invention;
FIG. 3 is a side elevational view of the surgical cannula depicted in FIG. 2;
FIG. 4 is a top plan view of the surgical cannula depicted in FIGS. 2 and 3 depicting an upper flange portion having apertures defined therein for insertion therethrough of a light, an irrigation tube, and/or a suction tube;
FIG. 5 is a side cross-sectional view of the surgical cannula depicted in FIGS. 2-4 inserted into a patient's body, in an undeployed position; and
FIG. 6 is a side cross-sectional view of the surgical cannula depicted in FIGS. 2-5 inserted into a patient's body, in a deployed position.

### DETAILED DESCRIPTION OF THE PREFERRED EMBODIMENT

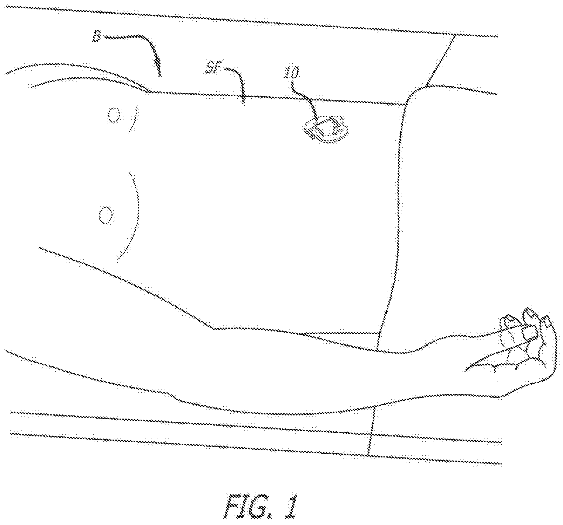

In accordance with the present invention, a surgical cannula 10, as depicted in FIG. 1, is provided for accessing a surgical site in the body B of a patient.

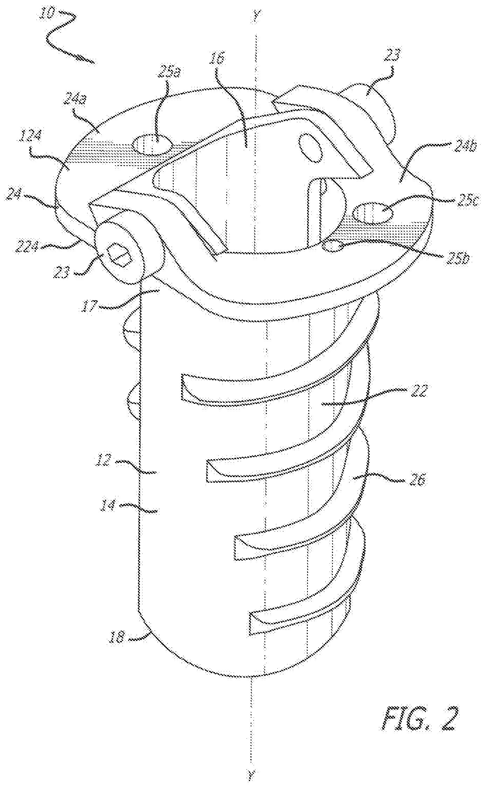

Referring to FIGS. 2, 3, 5, and 6, the surgical cannula 10 includes a cannula body 12 that can be substantially cylindrical, having a central longitudinal axis Y-Y and an external peripheral surface 14. The cannula body 12 has a bore 16, a proximal end 17, a distal end 18, and a length between the proximal end and the distal end. The bore 16 can be coaxial with central longitudinal axis Y-Y, and can extend centrally through the cannula body 12. The length is long enough such that the surgical cannula 10, when inserted into the patient's body B (FIG. 1), can extend from a superficial fascia SF of the patient's body B above the surgical site to at least adjacent the surgical site.

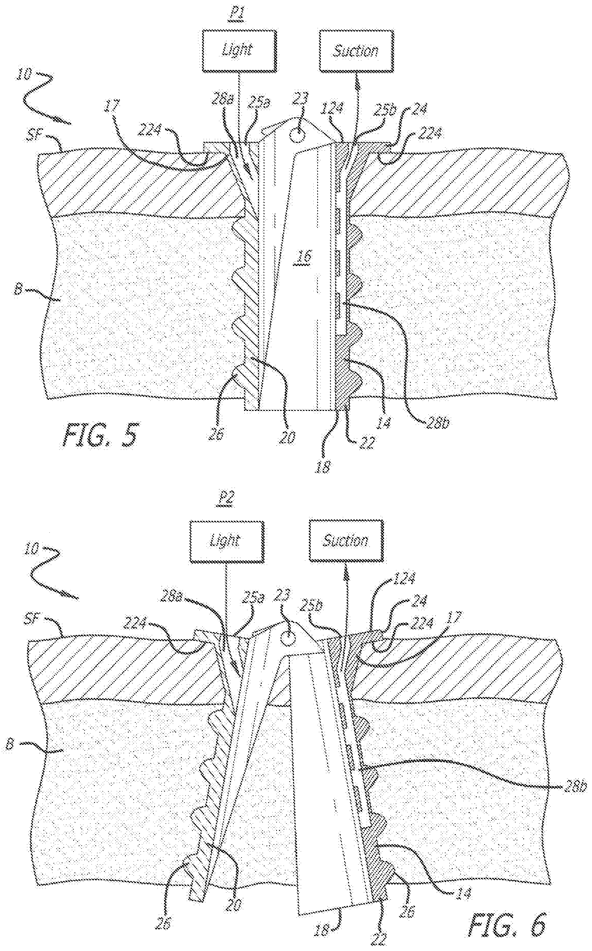

As depicted in FIGS. 2, 3, 5, and 6, the cannula body 12 defined by a first cannula body portion 20 and a second cannula body portion 22. The first and second cannula body portions 20 and 22 can be partially cylindrical, and can be pivotally connected to one another via a pair of opposed pivotal joints 23 adjacent the proximal end 17 of the cannula 10. In an undeployed position P1 (FIG. 5), the first cannula body portion 20 is partially received within the second cannula body portion 22.

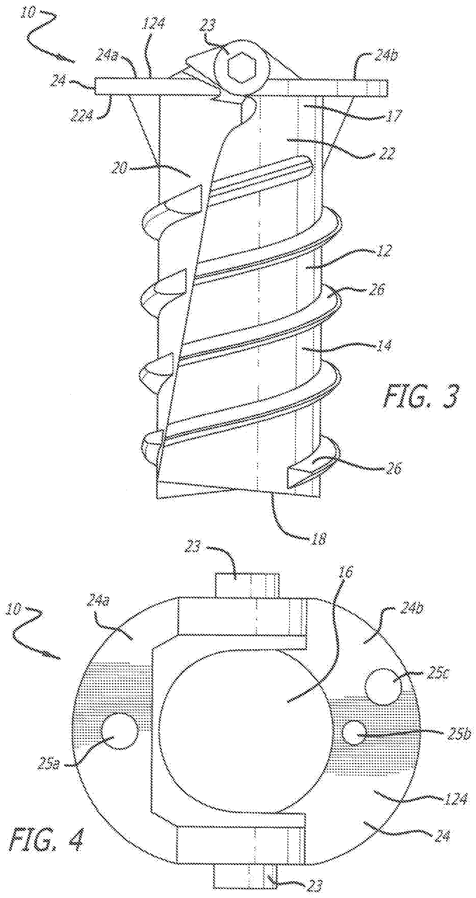

Referring to FIG. 3, a flange portion 24 is attached to the cannula body 12 at the proximal end 17. The bore 16 extends through flange portion 24 in addition to the cannula body 12. The flange portion 24, as depicted in FIG. 4, is divided into adjacent flange portions 24a and 24b attached to the first and second cannula body portions 20 and 22, respectively, and pivotal therewith. The flange portion 24 has defined therethrough at least one aperture. While, as depicted in FIG. 4, three apertures 25a, 25b, and 25c can be provided, the surgical cannula 10 is not limited to this number of apertures and can have fewer or more apertures. The flange portion 24 also has an upper surface 124 and a lower surface 224, with the apertures 25a, 25b, and 25c being defined between the upper surface 124 and the lower surface 224.

The surgical cannula 10 further includes a thread 26. Referring to FIGS. 3, 5, and 6, the thread 26 is provided around at least a portion of the external peripheral surface 14 between the proximal end 17 to the distal end 18.

At least one elongated channel is provided within the cannula body 12. While, as depicted in FIGS. 5 and 6, two channels 28a and 28b can be provided, the surgical cannula 10 is not limited to this number of channels and can have fewer or more channels. The at least one elongated channel (e.g., channels 28a and 28b) communicates with the at least one aperture (e.g., apertures 25a and 25b) in the flange portion 24, and extends downward from the flange portion 24 through the cannula body 12 toward the distal end 18. Preferably, the elongated channel (e.g., channels 28a and 28b) is substantially parallel to the central longitudinal axis Y-Y. Furthermore, the elongated channel (e.g., channels 28a and 28b) can be sized and shaped to accommodate placement of rigid and/or flexible instruments therethrough. For example, if substantially straight, the elongated channel (e.g., channels 28a and 28b can accommodate insertion of either rigid or flexible lights therethrough.

The surgical cannula 10 is inserted into the body B of the patient in the undeployed position P1, as depicted in FIG. 5, with the first and second cannula body portions 20 and 22 proximate to one another. In the undeployed position P1, the surgeon's access to the surgical site is via the bore 16, which provides an amount of access limited by the diameter of the bore 16. When the surgical cannula 10 of the present invention is fully inserted into the patient's body B, as depicted in FIG. 5, the lower surface 224 of the flange portion 24 contacts the superficial fascia SF, and acts as a stop, preventing the cannula 10 from being inadvertently inserted past the SF. In addition, the thread 26 acts to hold the cannula firmly in position in the patient's body B, without requiring the use of an external clamping arm, so the cannula 10 can be inserted with the patient under local sedation rather than full sedation. Moreover, the thread 26 will hold the cannula 10 in place in the patient's body B, even if the patient moves during the surgery.

When the surgical cannula 10 is inserted into the patient's body B, in the undeployed position P1, as depicted in FIG. 5, it can be moved to a deployed position P2, as depicted in FIG. 6, by pivoting apart the first and second cannula body portions 20 and 22 to a position spaced away from one another and spaced away from the central longitudinal axis Y-Y. By moving the cannula 10 to the deployed position P2, more space is created at the surgical site in which the surgeon can work with greater unimpeded access.

In addition, the apertures (e.g., apertures 25a and 25b), and the channels (e.g., channels 28a and 28b) in communication therewith, provide the surgeon with additional access to the surgical site in order to, for example, shine additional light on the surgical site, e.g., by inserting an LED attached to a cable therethrough. The surgeon also can insert a fiber optic cable through the apertures (e.g., apertures 25a and 25b) and the channels (e.g., channels 28a and 28b), to obtain a video image of the surgical site. In addition, the apertures (e.g., apertures 25a and 25b) and the channels (e.g., channels 28a and 28b) in communication therewith, provide the surgeon with a route to provide irrigation liquid to the surgical site, and/or apply suction to the surgical site to withdraw irrigation liquid, bone dust, blood, or other materials.

The presence of at least two apertures (e.g., apertures 25a and 25b), and at least two respective channels (e.g., channels 28a and 28b) in communication therewith, is advantageous in order to provide light to the surgical site via one aperture and channel combination, and to provide irrigation to the surgical site and draw suction from the surgical site, via another aperture and channel combination.

FIGS. 2 and 4 depict the surgical cannula 10 having three apertures 25a, 25b, and 25c, defined in the flange portion 24. The apertures 25a, 25b, and 25c are in communication with corresponding elongated channels 28a and 28b, and another channel (not shown) within the cannula body 12. Such a cannula is particularly advantageous because the surgeon can use one aperture/channel combination to provide light to the surgical site, use another aperture/channel combination to provide irrigation to the surgical site, and use yet another aperture/channel combination to draw suction on the surgical site.

Other embodiments of the invention will be apparent to those skilled in the art from consideration of the specification and practice of the invention disclosed herein. It is intended that the specification and examples be considered as exemplary only, with a true scope of the invention being indicated by the following claims.

## Claims

1. A surgical cannula (10) for accessing a surgical site in a body (B) of a patient, the surgical cannula (10), comprising:
a cannula body (12) having a proximal end (17), a distal end (18), and a peripheral surface (14), the cannula body (12) having a first cannula body portion (20) and a second cannula body portion (22), the first and second cannula body portions (20, 22) being attached to one another adjacent the proximal end (17), the cannula body (12) having a bore (16) extending from the proximal end (17) to the distal end (18), and a length between the proximal end (17) and the distal end (18) such that the cannula body (12) is adapted to extend from a superficial fascia (SF) of the patient above the surgical site to the surgical site;
a flange portion (24) attached to the cannula body (12) at the proximal end (17), the flange (24) having an upper surface (124) and a lower surface (224), and at least one aperture (25a, 25b) defined therebetween, the lower surface (224) being adapted to contact the superficial fascia (SF) of the body (B) of the patient above the surgical site;
a thread (26) around at least a portion of the peripheral surface (14) of the cannula body (12); and
at least one channel (28a, 28b) in communication with the at least one aperture (25a, 25b) in the flange (24), the at least one channel (28a, 28b) extending from the flange portion (24) through the cannula body (12) to at least adjacent the distal end (18) of the cannula body (12) such that the at least one channel (28a, 28b) is adapted to extend to at least adjacent the surgical site.

2. The surgical cannula (10) in accordance with claim 1, wherein the first and second cannula body portions (20, 22) are pivotally attached to one another with at least one pivotal joint (23) positioned at the proximal end (17), and adapted to allow the first and second cannula portions (20, 22) to pivot from an undeployed position (P1) proximate one another to a deployed position (P2) spaced apart from one another.

3. The surgical cannula (10) in accordance with claim 1, wherein the at least one aperture and the at least one channel in communication therewith are adapted to provide light to the surgical site.

4. The surgical cannula (10) in accordance with claim 1, wherein the at least one aperture (25a, 25b) and the at least one channel (28a, 28b) in communication therewith are adapted to provide irrigation to the surgical site.

5. The surgical cannula (10) in accordance with claim 1, wherein the at least one aperture (25a, 25b) and the at least one channel (28a, 28b) in communication therewith are adapted to provide suction of at least one of irrigation liquids and other materials from the surgical site.

6. The surgical cannula (10) in accordance with claim 1, wherein the at least one channel (28a, 28b) extends from the flange portion (24) through the cannula body (12) to at least adjacent the distal end (18) substantially parallel to the central longitudinal axis (Y-Y).

7. The surgical cannula (10) in accordance with claim 1, further comprising a plurality of apertures (25a, 25b, 25c) defined in the flange portion (24).

8. The surgical cannula (10) in accordance with claim 7, further comprising a plurality of elongated channels (28a, 28b), each channel of the plurality of channels (28a, 28b) being in communication with an aperture of the plurality of apertures (25a, 25b, 25c) in the flange portion (24), and each channel of the plurality of channels (28a, 28b) extending from the flange portion (24) to adjacent the distal end (18) of the cannula body (12).

9. The surgical cannula (10) in accordance with claim 8, wherein one of light, irrigation, and suction can be provided to the surgical site via each of the plurality of channels (28a, 28b).

## Patentansprüche

1. Eine chirurgische Kanüle (10) für einen Zugang zu einer Operationsstelle in einem Körper (B) eines Patienten, wobei die chirurgische Kanüle (10) aufweist:
einen Kanülenkörper (12), aufweisend ein proximales Ende (17), ein distales Ende (18) und eine Umfangsfläche (14), wobei der Kanülenkörper (12) einen ersten Kanülenkörperabschnitt (20) und einen zweiten Kanülenkörperabschnitt (22) aufweist, wobei der erste und der zweite Kanülenkörperabschnitt (20, 22) benachbart zu dem proximalen Ende (17) aneinander angebracht sind, wobei der Kanülenkörper (12) aufweist eine Bohrung (16), die sich vom proximalen Ende (17) zum distalen Ende (18) erstreckt, und eine Länge zwischen dem proximalen Ende (17) und dem distalen Ende (18), die derart ist, dass der Kanülenkörper (12) angepasst ist, um sich von einer oberflächlichen Faszie (SF) des Patienten über der Operationsstelle zu der Operationsstelle zu erstrecken,
einen Flanschabschnitt (24), der am proximalen Ende (17) am Kanülenkörper (12) angebracht ist, wobei der Flansch (24) eine obere Fläche (124) und eine untere Fläche (224) und mindestens eine dazwischen definierte Öffnung (25a, 25b) aufweist, wobei die untere Fläche (224) angepasst ist, um die oberflächliche Faszie (SF) des Körpers (B) des Patienten über der Operationsstelle zu kontaktieren,
ein Gewinde (26) um zumindest einen Abschnitt der Umfangsfläche (14) des Kanülenkörpers (12), und
mindestens einen Kanal (28a, 28b) in Kommunikation mit der mindestens einen Öffnung (25a, 25b) im Flansch (24), wobei sich der mindestens eine Kanal (28a, 28b) vom Flanschabschnitt (24) durch den Kanülenkörper (12) hindurch zu mindestens benachbart zu dem distalen Ende (18) des Kanülenkörpers (12) erstreckt, so dass der mindestens eine Kanal (28a, 28b) angepasst ist, um sich zu mindestens benachbart zu der Operationsstelle zu erstrecken.

2. Die chirurgische Kanüle (10) gemäß Anspruch 1, wobei der erste und der zweite Kanülenkörperabschnitt (20, 22) schwenkbar aneinander angebracht sind, mit mindestens einem Drehgelenk (23), das am proximalen Ende (17) positioniert ist und angepasst ist, um zu ermöglichen, dass der erste und der zweite Kanülenabschnitt (20, 22) aus einer Nicht-EntfaltungsPosition (P1) nahe beieinander in eine Entfaltungsposition (P2) im Abstand voneinander schwenken.

3. Die chirurgische Kanüle (10) gemäß Anspruch 1, wobei die mindestens eine Öffnung und der mindestens eine Kanal in Kommunikation damit angepasst sind, um der Operationsstelle Licht bereitzustellen.

4. Die chirurgische Kanüle (10) gemäß Anspruch 1, wobei die mindestens eine Öffnung (25a, 25b) und der mindestens eine Kanal (28a, 28b) in Kommunikation damit angepasst sind, um der Operationsstelle eine Spülung bereitzustellen.

5. Die chirurgische Kanüle (10) gemäß Anspruch 1, wobei die mindestens eine Öffnung (25a, 25b) und der mindestens eine Kanal (28a, 28b) in Kommunikation damit angepasst sind, um ein Absaugen von mindestens einem von Spülflüssigkeiten und anderen Materialien von der Operationsstelle bereitzustellen.

6. Die chirurgische Kanüle (10) gemäß Anspruch 1, wobei sich der mindestens eine Kanal (28a, 28b) von dem Flanschabschnitt (24) durch den Kanülenkörper (12) hindurch zu mindestens benachbart zu dem distalen Ende (18) im Wesentlichen parallel zu der zentralen Längsachse (Y-Y) erstreckt.

7. Die chirurgische Kanüle (10) gemäß Anspruch 1, ferner aufweisend eine Mehrzahl von Öffnungen (25a, 25b, 25c), die in dem Flanschabschnitt (24) definiert sind.

8. Die chirurgische Kanüle (10) gemäß Anspruch 7, ferner aufweisend eine Mehrzahl von langgestreckten Kanälen (28a, 28b), wobei jeder Kanal aus der Mehrzahl von Kanälen (28a, 28b) in Kommunikation mit einer Öffnung der Mehrzahl von Öffnungen (25a, 25b, 25c) im Flanschabschnitt (24) ist und sich jeder Kanal aus der Mehrzahl von Kanälen (28a, 28b) vom Flanschabschnitt (24) zu benachbart zum distalen Ende (18) des Kanülenkörpers (12) erstreckt.

9. Die chirurgische Kanüle (10) gemäß Anspruch 8, wobei der Operationsstelle über jeden von der Mehrzahl von Kanälen (28a, 28b) eines von Licht, Spülung und Absaugen bereitgestellt werden kann.

## Revendications

1. Une canule chirurgicale (10) pour accéder à un site chirurgical dans un corps (B) d'un patient, la canule chirurgicale (10) comprenant :
un corps de canule (12) présentant une extrémité proximale (17), une extrémité distale (18) et une surface périphérique (14), le corps de canule (12) présentant une première partie de corps de canule (20) et une deuxième partie de corps de canule (22), les première et deuxième parties de corps de canule (20, 22) étant fixées l'une à l'autre adjacent à l'extrémité proximale (17), le corps de canule (12) présentant un alésage (16) s'étendant de l'extrémité proximale (17) à l'extrémité distale (18), et une longueur entre l'extrémité proximale (17) et l'extrémité distale (18) de sorte que le corps de canule (12) est adapté pour s'étendre d'un fascia superficiel (SF) du patient au-dessus du site chirurgical au site chirurgical,
une partie de bride (24) fixée au corps de canule (12) à l'extrémité proximale (17), la bride (24) présentant une surface supérieure (124) et une surface inférieure (224), et au moins un orifice (25a, 25b) défini entre celles-ci, la surface inférieure (224) étant adaptée pour contacter le fascia superficiel (SF) du corps (B) du patient au-dessus du site chirurgical,
un filetage (26) autour au moins une partie de la surface périphérique (14) du corps de canule (12), et
au moins un canal (28a, 28b) en communication avec l'au moins un orifice (25a, 25b) dans la bride (24), l'au moins un canal (28a, 28b) s'étendant de la partie de bride (24) à travers le corps de canule (12) à au moins adjacent à l'extrémité distale (18) du corps de canule (12) de sorte que l'au moins un canal (28a, 28b) est adapté pour s'étendre à au moins adjacent au site chirurgicale.

2. La canule chirurgicale (10) selon la revendication 1, dans laquelle les première et deuxième parties de corps de canule (20, 22) sont fixées de manière pivotante l'une à l'autre avec au moins un joint pivotant (23) positionné à l'extrémité proximale (17), et adaptées pour permettre aux première et deuxième parties de canule (20, 22) de pivoter d'une position non-déployée (P1) proches l'une de l'autre à une position déployée (P2) espacées l'une de l'autre.

3. La canule chirurgicale (10) selon la revendication 1, dans laquelle l'au moins un orifice et l'au moins un canal en communication avec celui-ci sont adaptés pour fournir de la lumière au site chirurgical.

4. La canule chirurgicale (10) selon la revendication 1, dans laquelle l'au moins un orifice (25a, 25b) et l'au moins un canal (28a, 28b) en communication avec celui-ci sont adaptés pour fournir de l'irrigation au site chirurgical.

5. La canule chirurgicale (10) selon la revendication 1, dans laquelle l'au moins un orifice (25a, 25b) et l'au moins un canal (28a, 28b) en communication avec celui-ci sont adaptés pour fournir de l'aspiration d'au moins un parmi des liquides d'irrigation et d'autres matériaux du site chirurgical.

6. La canule chirurgicale (10) selon la revendication 1, dans laquelle l'au moins un canal (28a, 28b) s'étend de la partie de bride (24) à travers le corps de canule (12) à au moins adjacent à l'extrémité distale (18) essentiellement parallèle à l'axe central longitudinal (Y-Y).

7. La canule chirurgicale (10) selon la revendication 1, comprenant en outre une pluralité d'orifices (25a, 25b, 25c) définis dans la partie de bride (24).

8. La canule chirurgicale (10) selon la revendication 7, comprenant en outre une pluralité de canaux allongés (28a, 28b), chaque canal de la pluralité de canaux (28a, 28) étant en communication avec un orifice de la pluralité d'orifices (25a, 25b, 25c) dans la partie de bride (24), et chaque canal de la pluralité de canaux (28a, 28b) s'étendant de la partie de bride (24) à adjacent à l'extrémité distale (18) du corps de canule (12).

9. La canule chirurgicale (10) selon la revendication 8, dans laquelle une parmi la lumière, l'irrigation et l'aspiration peut être fournie au site chirurgical par chacun de la pluralité de canaux (28a, 28b).
